# EUROPÄISCHE PATENTSCHRIFT

(11) **EP 0 722 930 B1**
(45) Veröffentlichungstag und Bekanntmachung des Hinweises auf die Patenterteilung: **30.06.1999**
(21) Anmeldenummer: 96100120.3
(22) Anmeldetag: 05.01.1996
(51) Int. Cl.: C07C 68/02, C07C 69/96

(54) **Verfahren zur Herstellung von Arylcarbonaten**
Process for producing aryl carbonates
Procédé de fabrication de carbonates d'aryle

(30) Priorität: 18.01.1995 DE 19501364
(43) Veröffentlichungstag der Anmeldung: 24.07.1996
(73) Patentinhaber: BAYER AG, 51368 Leverkusen (DE)
(72) Erfinder: Ooms, Pieter, Dr., D-47800 Krefeld (DE); Buysch, Hans-Josef, Dr., D-47809 Krefeld (DE)

(56) Entgegenhaltungen:
- US-A- 5 239 105

## Beschreibung

Die Erfindung betrifft ein Verfahren zur Herstellung von Carbonaten mit aromatischen Estergruppen durch Umsetzung von aromatischen Monohydroxyverbindungen mit Phosgen oder Chlorkohlensäureestern aromatischer Monohydroxyverbindungen unter Abspaltung von Chlorwasserstoff in Gegenwart von Hartstoffen mit metallartigen Eigenschaften (Keramikvorprodukte) als heterogene Katalysatoren.

Carbonate mit aromatischen Estergruppen eignen sich zur Herstellung von Polycarbonaten nach dem Schmelzumesterungsverfahren, zur Herstellung von Phenylurethanen oder sind Vorprodukte von Wirkstoffen aus dem Pharma- und Pflanzenschutzsektor.

Es ist bekannt, daß Arylcarbonate durch Phasengrenzflächenphosgenierung (Schotten-Baumann-Reaktion) von aromatischen Hydroxyverbindungen gewonnen werden können. Dabei wirkt sich die Verwendung von Lösungsmitteln und Natronlauge nachteilig aus, da durch die wässrige Lauge eine teilweise Verseifung von Phosgen oder Chlorkohlensäureester stattfinden kann. In jedem Falle werden große Mengen Kochsalz als Nebenprodukt erhalten. Ferner muß Sorge für die Lösungsmittel-Rückgewinnung getragen werden.

Man hat daher eine Kondensation ohne Mitverwendung von Lösungsmitteln in Gegenwart von Tetramethylammoniumhalogeniden als Katalysatoren vorgeschlagen (US-A-2 837 555). Dabei sind daher die benötigten Katalysatormengen relativ groß. Man muß in der Regel mit 5 bis 7 Gew.-% Katalysator, bezogen auf die eingesetzte Phenolmenge, arbeiten, um wirtschaftliche Reaktionsgeschwindigkeiten zu erhalten; die Reaktionstemperaturen von 180°C bis 215°C bringen die Gefahr einer Zersetzung der thermolabilen Tetramethylammoniumhalogenide mit sich. Der Katalysator muß ferner anschließend durch Waschen mit Wasser entfernt werden, wodurch seine Rückgewinnung erheblich erschwert wird. Darüber hinaus wird weit mehr als die stöchiometrisch notwendige Menge an Phosgen verbraucht.

Nach einem weiteren Verfahren (US-A-3 234 263) werden Diarylcarbonate durch Erhitzen von Phenylchlorkohlensäureestern in Gegenwart großer Mengen (Erd)alkaliverbindungen mit tertiären Stickstoffbasen als Katalysatoren erhalten. Dieses Verfahren hat jedoch den Nachteil, daß man hohe Temperaturen anwenden und die Katalysatoren wie (Erd)Alkaliverbindungen teilweise lösen muß, um auch nur annähernd auf wirtschaftlich. vertretbare Reaktionszeiten zu kommen. Bei diesem Verfahren geht die Hälfte des ursprünglich eingesetzten Phosgens in Form von CO₂ verloren. Außerdem muß man in einem vorgelagerten separaten Verfahrensschritt die Chlorkohlensäureester synthetisieren.

Nach der US-A-2 362 865 erhält man Diarylcarbonate durch Phosgenierung von aromatischen Hydroxyverbindungen in Gegenwart von metallischem Titan, Eisen, Zink und Zinn oder in Form ihrer löslichen Salze, besonders der Chloride und Phenolate. Obwohl sehr gute Ausbeuten erhalten werden, ist es schwierig, die Katalysatoren von den Produkten zu trennen. Man muß sogar bei Destillationen mit einer gewissen Flüchtigkeit dieser Verbindungen und auch mit thermischen Zersetzungen durch diese Verbindungen rechnen, die zu Verunreinigungen, Qualitätsminderungen und Ausbeuteeinbußen führen.

In US-A-5 239 105 werden Oxide von V, Nb, Ta und W sowie silikatische Materialien als Katalysatoren für die Herstellung von Diarylcarbonaten vorgeschlagen. Die Selektivität der Bildung von Diarylcarbonat ist bei Einsatz dieser Katalysatoren jedoch noch unbefriedigend.

Somit erscheint es sinnvoll, heterogene, nicht lösliche Katalysatoren zu verwenden, die eine Aufarbeitung des Reaktionsgemisches wesentlich erleichtern. Auch dazu wurden Vorschläge gemacht. So wird nach der Lehre der EP-A-516 355 vor allem Aluminiumtrifluorid empfohlen, das gegebenenfalls auf Träger wie Alumosilikate aufgebracht wird. Die Synthese von Aluminiumfluorid ist jedoch durch die Handhabung von Fluor oder Flußsäure sehr aufwendig und teurer. Weiter werden in der WO 91/06526 Metallsalze auf porösen Trägern als Katalysatoren für die erfindungsgemäße Umsetzungen beschrieben. Wie aus den Versuchsbeispielen hervorgeht, ist eine vollkontinuierliche Phosgenierung von Phenol an solchen Katalysatoren nur in der Gasphase möglich, was aber relativ hohe Reaktionstemperaturen und die Gefahr der Zersetzung der empfindlichen Chlorameisensäureester mit sich bringt. Offensichtlich ist eine Phosgenierung von Phenol mit diesen Katalysatoren in der Flüssigphase nicht durchführbar, da das heiße, flüssige Phenol die aktiven Katalysatorbestandteile auswäscht.

Die Aufgabe der Erfindung bestand also darin, einfacher zugängliche, wirksame heterogene Katalysatoren für die Herstellung von Diarylcarbonaten zu entwickeln.

Es wurde nun gefunden, daß Verbindungen der allgemeinen Formel (III) wie beispielsweise Titancarbonitrid oder Zirkonborid ausgezeichnete Katalysatoren für die Umsetzung von Phosgen oder Chlorkohlensäureestern mit aromatischen Hydroxyverbindungen darstellen. Dies ist besonders überraschend und unerwartet, weil nach der vorgängigen Lehre der WO 91/06526 Oxide von Metallen wie Titan und Zirkon bevorzugt als widerstandsfähige und inerte Trägermaterialien genannt werden.

Der Gegenstand der Erfindung ist ein Verfahren zur Herstellung von Arylcarbonaten durch Umsetzung von aromatischen Monohydroxyverbindungen mit Phosgen oder Chlorameisensäureestern aromatischer Monohydroxyverbindungen, dadurch gekennzeichnet, daß man bei Temperaturen im Bereich von 50 bis 450°C, gegebenenfalls bei einem Druck von 0,05 bis 20 bar in Gegenwart von Verbindungen der allgemeinen Formel (III)

AₓB_{y}C_{z}D_{w} (III),

in welcher
- A: für ein Element aus den Gruppen 3 bis 10, 13 und 14 des periodischen Systems der Elemente (IUPAC-Notation) steht und
- B: für ein Element aus den Gruppen 13, 14, 15 und 16 mit Ausnahme von Sauerstoff steht und
- C: für ein Element aus den Gruppen 14 und 15 steht und
- D: für ein Element aus den Gruppen 14 und 15 steht und
- x: für eine Zahl von 1 bis 4 steht und
- y: für eine Zahl von 1 bis 4 steht und
- z: für eine Zahl von 0 bis 4 steht und
- w: für eine Zahl von 0 bis 4 steht,
als heterogene Katalysatoren, wobei A, B, C und D jeweils aus verschiedenen Gruppen oder bei gleicher Gruppe aus verschiedenen Perioden stammen, mit der Maßgabe, daß A verschieden von Aluminium ist, wenn B Kohlenstoff ist und gleichzeitig z und w gleich 0 sind, arbeitet.

Das erfindungsgemäße Verfahren hat den großen Vorteil, daß man den Katalysator sehr leicht abtrennen kann und keine Verunreinigungen im rohen Reaktionsprodukt verbleiben. Die Aufarbeitung wird dadurch wesentlich vereinfacht.

Aromatische Monohydroxyverbindungen für das erfindungsgemäße Verfahren sind solche der Formel

Ar¹-OH (I),

worin
- Ar¹: Phenyl, Naphthyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen und heterocyclischen Reste durch 1 oder 2 Substituenten wie geradkettiges oder verzweigtes C₁-C₄-Alkyl, geradkettiges oder verzweigtes C₁-C₄-Alkoxy, die mit Phenyl, Cyano und Halogen (z.B. F, Cl, Br) substituiert sein können und wobei weiterhin die heterocyclischen Reste mit einem ankondensierten Benzolkern verbunden sein können.

Beispiele für aromatische Monohydroxyverbindungen der Formel (I) sind: Phenol, o-, m- und p-Kresol, o-, m- und p-Isopropylphenol, die entsprechenden Halogen- bzw. Alkoxyphenole, wie p-Chlorphenol bzw. p-Methoxyphenol, ferner Monohydroxyverbindungen des Naphthalins, Anthracens und Phenanthrens, weiterhin 4-Hydroxy-pyridin und Hydroxychinoline. Vorzugsweise werden gegebenenfalls substituierte Phenole, ganz besonders bevorzugt Phenol selbst eingesetzt.

Das erfindungsgemäße Verfahren kann sowohl mit Phosgen als auch mit Chlorkohlensäureestern aromatischer Monohydroxyverbindungen durchgeführt werden. Für den Fall der Durchführung mit Phosgen entsteht zunächst der Chlorkohlensäureester, der mit weiterer im Reaktionsgemisch vorhandener aromatischer Monohydroxyverbindung zum Diarylcarbonat umgesetzt wird.

Geht man von Chlorkohlensäureestern und einer aromatischen Monohydroxyverbindung aus, können symmetrische oder unsymmetrische Carbonate erhalten werden.

Geeignete aromatische Chlorkohlensäureester für das erfindungsgemäße Verfahren sind solche der Formel (II)

Ar¹-OCOCl (II),

worin
- Ar¹: die bei Formel (I) angegebene Bedeutung hat.

Geeignete Katalysatoren im Sinne der Erfindung sind Verbindungen der allgemeinen Formel (III)

AₓB_{y}C_{z}D_{w} (III),

in welcher
- A: für ein Element aus den Gruppen 3 bis 10, 13 und 14 des periodischen Systems der Elemente (IUPAC-Notation) steht und
- B: für ein Element aus den Gruppen 13, 14, 15 und 16 mit Ausnahme von Sauerstoff steht und
- C: für ein Element aus den Gruppen 14 und 15 steht und
- D: für ein Element aus den Gruppen 14 und 15 steht und
- x: für eine Zahl von 1 bis 4 steht und
- y: für eine Zahl von 1 bis 4 steht und
- z: für eine Zahl von 0 bis 4 steht und
- w: für eine Zahl von 0 bis 4 steht.

Als Beispiele für A seien genannt:
Ti, Zr, Hf, V, Nb, Ta, Cr, Mo, W, Y, La, Al, Ga, In, Tl, Mn, Tc, Re, Fe, Co, Ni, Sn, Pb, Ge, bevorzugt sind Ti, Zr, Hf, V, Nb, Ta, Cr, Mo, W, Y, La, Al, Si, Sn. Mn, Fe, Ni, besonders bevorzugt sind Ti, Zr, V, Nb, Ta, Mo, W, Al und Si.

Als Beispiele für B seien genannt:
B, C, Si, N, P, Se und Te, bevorzugt sind B, C, Si, N, P und Te, besonders bevorzugt sind B, C, Si, N und P.

Als Beispiele für C seien genannt:
C und N.

Als Beispiele für D seien genannt:
C und N.

In der Formel (III) können eine oder mehrere Elemente A, B, C oder D auch in verschiedener Wertigkeit nebeneinander vorkommen.

Die Verbindungen der Formel (III) können aus verschiedenen Vorprodukten, beispielsweise Metallsalzen, Metalloxiden und Metallen hergestellt werden.

Diese Verbindungen und Herstellverfahren für solche Verbindungen sind beispielsweise in Kirk-Othmer, Encyclopedia of Chemical Technology, 3. Auflage, Bd. 20, S. 414 ff, S. 658, Bd. 21, S. 169 ff, Bd. 22, S. 355 ff, S. 622 ff., S. 659 ff., Bd. 13, S. 814 ff., New York 1969/1983, Ullmann's Encyclopedia of industrial chemistry, 5. Aufl.., Bd. A5, S. 61 ff., Bd. 17, S. 429 ff., Bd. A17, S. 341 ff, Weinheim 1966/1991 beschrieben.

Besonders geeignet als heterogene Katalysatoren sind Hartstoffe mit metallartigen Eigenschaften (Keramikvorprodukte).

Sie können in kristalliner Form in verschiedenen Modifikationen vorliegen. Sie können ganz oder teilweise amorph sein.

Bei den im erfindungsgemäßen Verfahren einsetzbaren Katalysatoren handelt es sich bevorzugt um Carbide, Nitride, Carbonitride, Boride, Borcarbonitride, Phosphide, Silizide und Phosphornitride, beispielsweise Titancarbid, Titannitrid, Titancarbonitrid, Titanmonoborid, Titandiborid, Titansilizide, wie Titandisilizide, Zirkoncarbid, Zirkonnitrid, Zirkoncarbonitrid, Zirkondiborid, Zirkonsilizid, Zirkonphosphid, Hafniumnitrid, Wolframcarbid, Diwolframcarbid, Wolframnitrid, Diwolframnitrid, Wolframborid, Diwolframborid, Diwolframpentaborid, Wolframdisilizide, Diwolframtrisilizide, Pentawolframtrisilizide, Vanadiumcarbid, Divanadiumcarbid, Vanadiumnitrid, Vanadiumborid, Vanadiumdiborid, Vanadiumsilizid, Divanadiumsilizid, Vanadiumphosphid, Vanadiumdiphosphid, Divanadiumphosphid, Trivanadiumphosphid, Vanadiumselenid, Vanadiumtellurid, Vanadiumarsenid, Niobcarbid, Niobnitrid, Tantalnitrid, Ditantalnitrid, Tantalcarbid, Tantalborid, Dimolybdännitrid, Lanthanhexaborid, Yttriumhexaborid, Chromnitrid, Mangannitrid, Tellurnitrid, Rheniumnitrid, Eisennitrid, Cobaltnitrid, Nickelnitrid, Bornitrid, Aluminiumnitrid, Galliumnitrid, Indiumnitrid, Tellurnitrid, Siliziumnitrid, Germaniumnitrid, weiterhin ternäre Substanzen, beispielsweise Tantalzirkoncarbid, Tantalhafniumcarbid, Niobwolframcarbide und quaternäre Substanzen, beispielsweise Titanborcarbonitrid, Titanphosphorcarbonitrid, Zirkonborcarbonitrid.

Die Katalysatoren im Sinne der Erfindung können getrocknet, wasserfrei, teilgetrocknet oder ungetrocknet eingesetzt werden.

Bevorzugte Katalysatoren besitzen BET-Oberflächen von 0,1 bis 500 m²/g, besonders bevorzugt solche von 0,5 bis 200 m²/g und ganz besonders bevorzugt solche von 1 bis 100 m²/g. Es können saure, neutrale und basische Katalysatoren verwendet werden.

Die Katalysatoren können z.B. als Pulver oder Formkörper eingesetzt werden und nach der Umsetzung z.B. durch Filtration, Sedimentation oder Zentrifugieren abgetrennt werden. Für den Fall der Anordnung als Festbett werden die Katalysatoren vorzugsweise als Formkörper, z.B. als Kugeln, Zylinder, Stäbchen, Hohlzylinder, Ringe usw. eingesetzt.

Die Katalysatoren werden beim Arbeiten mit suspendiertem Katalysator in Rührgefäßen oder Blasensäulen in Mengen von 0,5 bis 100 Gew.-%, bevorzugt von 5 bis 100 Gew.-% und besonders bevorzugt von 5 bis 50 Gew.-%, bezogen auf die eingesetzte Menge an Monohydroxyverbindung, verwendet.

Im Falle einer kontinuierlichen Fahrweise im Gegen- oder Gleichstrom oder in der Rieselphase oder in der Gasphase am Festbettkatalysator werden Katalysatorbelastungen von 0,1 bis 20 g aromatische Hydroxyverbindung pro g Katalysator pro Stunde, bevorzugt 0,2 bis 10 g · g⁻¹ · h⁻¹ und besonders bevorzugt von 0,2 bis 5 g · g⁻¹ · h⁻¹ verwendet.

Die in diskontinuierlichen Versuchen verwendeten Katalysatoren können bei gleichen Einsatzstoffen ohne Reinigung wiederholt eingesetzt werden. Bei einem Wechsel der Einsatzstoffe werden die Katalysatoren zweckmäßig durch Extrahieren mit inerten Lösungsmitteln, wie sie beispielsweise weiter unten als Reaktionsmedien genannt sind, oder mit Alkoholen, wie Methanol, Ethanol, Isopropanol oder Butanol, mit Estern oder Amiden der Essigsäure oder durch Behandlung mit überhitztem Wasserdampf oder Luft gereinigt.

Bei kontinuierlicher Arbeitsweise können die eingesetzten Katalysatoren über lange Zeit im Reaktor verbleiben. Eine Regenerierung kann gegebenenfalls durch Überleiten von überhitztem Wasserdampf gegebenenfalls unter Zugabe von untergeordneten Mengen an Luft (etwa 0,1 bis 20 Gew.-%, bezogen auf die eingesetzte Wasserdampfmenge) bei 150 bis 800°C oder durch Überleiten von 0,01 bis 20 Gew.-% Sauerstoff enthaltenden Verdünnungsgasen wie Stickstoff, oder Kohlendioxid oder durch Kohlendioxid allein bei 200 bis 800°C erfolgen. Die bevorzugte Regenerierungstemperatur liegt bei 150 bis 700°C, besonders bevorzugt bei 200 bis 600°C.

Das erfindungsgemäße Verfahren wird bei einer Temperatur im Bereich von 50 bis 450°C, bevorzugt 100 bis 400°C, besonders bevorzugt 100 bis 350°C durchgeführt. Während der Durchführung des erfindungsgemäßen Verfahrens kann die Temperatur im genannten Bereich verändert, in bevorzugter Weise erhöht werden.

Das erfindungsgemäße Verfahren wird bei einem Druck von 0,05 bis 20 bar, vorzugsweise 1 bis 5 bar, durchgeführt.

Man kann das erfindungsgemäße Verfahren unter Mitwirkung von Lösungsmitteln wie aliphatischen und aromatischen Kohlenwasserstoffen, wie Pentan, Hexan, Octan, Benzol, isomeren Xylolen, Diethylbenzol, Alkylnaphthalinen, Biphenyl; halogenierten Kohlenwasserstoffen, wie Dichlormethan, Trichlorethylen usw. durchführen.

Man kann das erfindungsgemäße Verfahren sowohl in der Gasphase als auch in der Flüssigphase durchführen.

Vorzugsweise wird das Verfahren in der Schmelze durchgeführt, beispielsweise, indem man in eine Suspension eines Katalysators in einer Schmelze der aromatischen Monohydroxyverbindung der Formel (I) Phosgen oder einen Chlorkohlensäureester der Formel (II) einleitet und nach Beendigung der Reaktion den Katalysator z.B. durch Filtration oder Zentrifugieren abtrennt.

Das Verfahren wird in der Gasphase durchgeführt, indem man Phosgen und Phenol verdampft und das Gemisch über ein Bett eines in einem Rohr angeordneten stückigen Katalysators leitet.

Eine weitere bevorzugte Ausführungsform der Synthese ist die Begasung einer Schmelze der aromatischen Monohydroxyverbindung der Formel (I) mit darin suspendiertem Katalysator mit Phosgen oder Phosgen-Chlorwasserstoff-Gemischen oder mit Chlorkohlensäureestern der Formel (II) in einer kontinuierlich arbeitenden Blasensäule bzw. Blasensäulen-Kaskade.

Eine weitere bevorzugte Durchführungsform ist das Gleichstromverfahren, bei dem aromatische Hydroxyverbindungen der Formel (I) und Phosgen bzw. Chlorkohlensäureester der Formel (II) im Gleichstrom beispielsweise in der Rieselphase von oben her auf eine in einem Rohr angeordnete Katalysatorschüttung aufgebracht und unten am Fuß des Rohres Chlorwasserstoff und Phosgenierungsprodukte abgezogen werden.

Eine weitere bevorzugte Ausführungsform mit besonders günstigen Ergebnissen ist die Durchführung der erfindungsgemäßen Umsetzung im Gegenstrom in der Rieselphase, wobei die aromatische Monohydroxyverbindung der Formel (I) als Schmelze oder in Form einer Lösung oben auf ein Katalysator-Bett gegeben wird und diesem Flüssigkeitstrom von unten ein Strom von Phosgen oder Chlorkohlensäureester entgegengeschickt wird. Zweckmäßig wird diese Ausführungsform in einem senkrecht stehenden Rohrreaktor durchgeführt, die auch Zwischenböden zur verbesserten Verteilung von Gas- und Flüssigkeitsstrom enthalten kann.

Eine weitere bevorzugte Ausführungsform ist das Gasphasenverfahren bei Temperaturen von 150 bis 450°C, bevorzugt 180 bis 350°C mit Drucken von 0,05 bis 2, bevorzugt 0,1 bis 0,8 bar.

Bei diesem Verfahren wird der Druck so mit der Temperatur variiert, daß die Komponenten in der Gasphase bleiben und nicht auf der Katalysatorschüttung kondensieren.

Das molare Verhältnis der Reaktionspartner aromatische Monohydroxyverbindungen der Formel (I) zu Phosgen beträgt 0,5 bis 8:1, bevorzugt 1,5 bis 3:1. Das äquivalente molare Verhältnis ist in diesem Fall 2:1.

In entsprechender Weise wird die aromatische Monohydroxyverbindung mit einem Chlorkohlensäureester im Molverhältnis von 0,25 bis 4:1, bevorzugt 0,8 bis 1,5:1 umgesetzt. In diesem Fall beträgt das molare Verhältnis 1:1.

Das durch heterogene Katalyse gewonnene rohe aromatische Carbonat ist häufig schon sehr rein und kann nach Entgasung von restlichem Chlorwasserstoff oder anderen flüchtigen Stoffen bereits in dieser Form für viele Zwecke verwendet werden. Für anspruchsvollere Anwendungen kann das Carbonat gegebenenfalls z.B. durch Destillation oder Kristallisation weiter gereinigt werden.

### Beispiele

- Fa. H.C. Starck =: H.C. Starck GmbH & Co. KG, Im Schleeke 78-91, D-38642 Goslar
- Fa. Strem =: Strem Chemicals, Inc. 7 Mulliken Way, Dexter Industrial Park Newburyport, MA 01950, U.S.A.

### Beispiel 1

In einem Planschlifftopf mit Strombrechern, einem Begasungsrührer und Rückflußkühler wurden 141 g (1,50 mol) Phenol in Gegenwart von 14,1 g (10 Gew.-% bezogen auf Phenol) eines pulverförmigen Titancarbids der Fa. H.C. Starck bei 140°C mit 0,42 mol/h Phosgen kontinuierlich begast. Nach etwa 2 h Reaktionszeit betrug der Phenolumsatz 18,2 %, wobei 0,04 g Chlorameisensäurephenylester und 28,7 g Diphenylcarbonat gebildet wurde. Die Selektivität zu Kohlensäureestern war ca. 99 %.

### Beispiel 2

Das Beispiel 1 wurde mit 14,1 g eines pulverförmigen Titannitrids der Fa. H.C. Starck bei 140°C wiederholt. Nach 2 h Reaktionszeit betrug der Phenolumsatz 21,6 %, wobei 0,03 g Chlorameisensäurephenylester und 34,3 g Diphenylcarbonat gebildet wurden. Die Selektivität zu Kohlensäureestern war ca. 99 %.

### Beispiel 3

Das Beispiel 1 wurde mit 14,1 g eines pulverförmigen Titancarbonitrids der Fa. H.C. Starck bei 140°C wiederholt. Nach 2 h Reaktionszeit betrug der Phenolumsatz 32,6 %, wobei 52,1 g Diphenylcarbonat gebildet wurde. Die Selektivität war größer als 99 %.

### Beispiel 4

Das Beispiel 1 wurde mit 14,1 g eines pulverförmigen Titandiborids der Fa. H.C. Starck bei 140°C wiederholt. Nach 2 h Reaktionszeit betrug der Phenolumsatz 27,7 %, wobei 0,06 g Chlorameisensäurephenylester und 43,9 g Diphenylcarbonat gebildet wurden. Die Selektivität zu Kohlensäureestern war ca. 99 %.

### Beispiel 5

Das Beispiel 1 wurde mit 14,1 eines Titandisilizids der Fa. Strem bei 140°C wiederholt. Nach 2 h Reaktionszeit betrug der Phenolumsatz 14,7 %, wobei 23,4 g Diphenylcarbonat gebildet wurde. Die Selektivität war größer als 99 %.

### Beispiel 6

Das Beispiel 1 wurde mit 14,1 g eines pulverförmigen Zirkonnitrids der Fa. H.C. Starck bei 140°C wiederholt. Nach 2 h Reaktionszeit betrug der Phenolumsatz 9,4 %, wobei 2,2 g Chlorameisensäurephenylester und 12,5 g Diphenylcarbonat gebildet wurden. Die Selektivität zu Kohlensäureestern war 92,4 %.

### Beispiel 7

Das Beispiel 1 wurde mit 14,1 g eines pulverförmigen Zirkondiborids der Fa. H.C. Starck bei 140°C wiederholt. Nach 2 h Reaktionszeit betrug der Phenolumsatz 35,7 %, wobei 0,4 g Chlorameisensäurephenylester und 54,9 g Diphenylcarbonat gebildet wurden. Die Selektivität zu Kohlensäureestern war größer als 96 %.

### Beispiel 8

Das Beispiel 1 wurde mit 14,1 g eines pulverförmigen Aluminiumnitrids der Fa. H.C. Starck bei 140°C wiederholt. Nach 2 h Reaktionszeit betrug der Phenolumsatz 29,3 %, wobei 46,4 g Diphenylcarbonat gebildet wurde. Die Selektivität war größer als 98 %.

### Beispiel 9

Das Beispiel 1 wurde mit 14,1 g eines pulverförmigen Vanadiumcarbids der Fa. H.C. Starck bei 140°C wiederholt. Nach 2 h Reaktionszeit betrug der Phenolumsatz 16,3 %, wobei 0,3 g Chlorameisensäurephenylester und 25,5 g Diphenylcarbonat gebildet wurde. Die Selektivität zu Kohlensäureestern war 98,2 %.

### Beispiel 10

Das Beispiel 1 wurde mit 14,1 g eines pulverförmigen Molybdändisilizids der Fa. H.C. Starck bei 140°C wiederholt. Nach 2 h Reaktionszeit betrug der Phenolumsatz 8,7 %, wobei 4,2 g Chlorameisensäurephenylester und 10,3 g Diphenylcarbonat gebildet wurden. Die Selektivität zu Kohlensäureestern war ca. 95 %.

### Beispiel 11

Das Beispiel 1 wurde mit 14,1 g eines Dimolybdäncarbids der Fa. H.C. Starck bei 140°C wiederholt. Nach 2 h Reaktionszeit betrug der Phenolumsatz 6,0 %, wobei 5,1 g Chlorameisensäurephenylester und 5,8 g Diphenylcarbonat gebildet wurde. Die Selektivität zu Kohlensäureestern war 97,3 %.

### Beispiel 12

Das Beispiel 1 wurde mit 14,1 g eines pulverförmigen Wolframmonoborids der Fa. Strem bei 140°C wiederholt. Nach 2 h Reaktionszeit betrug der Phenolumsatz 17,3 %, wobei 4,8 g Chlorameisensäurephenylester und 24,2 g Diphenylcarbonat gebildet wurde. Die Selektivität zu Kohlensäureestern war ca. 99 %.

### Beispiel 13

Das Beispiel 1 wurde mit 14,1 g eines pulverförmigen Diwolframborids der Fa. Strem bei 140°C wiederholt. Nach 2 h Reaktionszeit betrug der Phenolumsatz 8,8 %, wobei 4,7 g Chlorameisensäurephenylester und 10,7 g Diphenylcarbonat gebildet wurde. Die Selektivität zu Kohlensäureestern war ca. 98 %.

### Beispiel 14 (zum Vergleich)

Das Beispiel 1 wurde ohne Zusatz von Katalysator bei 140°C wiederholt. Nach 2 h Reaktionszeit betrug der Phenolumsatz weniger als 0,2 %.

### Beispiel 15

In einem Dreihalskolben mit Thermometer und Rückflußkühler erhitzt man ein Gemisch aus 9,4 g (0,10 mol) Phenol und 15,7 g (0,10 mol) Chlorameisensäurephenylester in Gegenwart von 0,94 g (10 Gew.-% bezogen auf Phenol) eines pulverförmigen Titandiborids der Fa. H.C. Starck auf 100°C. Nach 5 h Reaktionszeit wird ein Phenolumsatz von 68,6 % zu Diphenylcarbonat gefunden. Die Carbonatselektivität war >99 %.

### Beispiel 16

Das Beispiel 15 wurde mit demselben Katalysator bei 120°C wiederholt. Nach 3 h Reaktionszeit betrug der Phenolumsatz zu Diphenylcarbonat 87,0 %. Die Carbonatselektivität war >99 %.

### Beispiel 17

Das Beispiel 15 wurde mit demselben Katalysator bei 140°C wiederholt. Nach 1 h Reaktionszeit betrug der Phenolumsatz zu Diphenylcarbonat 76,5 %. Die Carbonatselektivität war >99 %.

### Beispiel 18

Das Beispiel 15 wurde mit demselben Katalysator bei 160°C wiederholt. Nach 1 h Reaktionszeit betrug der Phenolumsatz zu Diphenylcarbonat 99 %. Die Carbonatselektivität war >99 %.

### Beispiel 19

Das Beispiel 15 wurde mit 0,94 g eines pulverförmigen Titancarbids der Fa. H.C. Starck bei 160°C wiederholt. Nach 1 h Reaktionszeit betrug der Phenolumsatz zu Diphenylcarbonat 80,7 %. Die Carbonatselektivität war >99 %.

### Beispiel 20

Das Beispiel 15 wurde mit 0,94 g eines Titannitrids der Fa. H.C. Starck bei 160°C wiederholt. Nach 1/2 h Reaktionszeit betrug der Phenolumsatz zu Diphenylcarbonat 80,0 %. Die Carbonatselektivität war >99 %.

### Beispiel 21

Das Beispiel 15 wurde mit 0,94 g eines pulverförmigen Titancarbonitrids der Fa. H.C. Starck bei 160°C wiederholt. Nach 1/2 h Reaktionszeit betrug der Phenolumsatz zu Diphenylcarbonat 94,1 %. Die Carbonatselektivität war >99 %.

### Beispiel 22

Das Beispiel 15 wurde mit 0,94 g eines pulverförmigen Zirkondiborids der Fa. H.C. Starck bei 140°C wiederholt. Nach 1 h Reaktionszeit betrug der Phenolumsatz zu Diphenylcarbonat 86,5 %. Die Carbonatselektivität war >99 %.

### Beispiel 23

Das Beispiel 15 wurde mit 0,94 g eines pulverförmigen Vanadiumcarbids der Fa. H.C. Starck bei 160°C wiederholt. Nach 1 h Reaktionszeit betrug der Phenolumsatz zu Diphenylcarbonat 84,4 %. Die Carbonatselektivität war >99 %.

### Beispiel 24

Das Beispiel 15 wurde mit 0,94 g eines pulverförmigen Wolframmonoborids der Fa. Strem bei 160°C wiederholt. Nach 5 h Reaktionszeit betrug der Phenolumsatz zu Diphenylcarbonat 74,4 %. Die Carbonatselektivität war >99 %.

## Patentansprüche

1. Verfahren zur Herstellung von Arylcarbonaten durch Umsetzung von aromatischen Monohydroxyverbindungen mit Phosgen oder Chlorkohlensäureestern aromatischer Monohydroxyverbindungen, dadurch gekennzeichnet, daß man die Umsetzung bei einer Temperatur im Bereich von 50 bis 450°C, bei einem Druck von 0,05 bis 20 bar in Gegenwart von einem oder mehreren Verbindungen der allgemeinen Formel (III)
AₓB_{y}C_{z}D_{w} (III),
in welcher
A für ein Element aus den Gruppen 3 bis 10, 13 und 14 des periodischen Systems der Elemente (IUPAC-Notation) steht und
B für ein Element aus den Gruppen 13, 14, 15 und 16 mit Ausnahme von Sauerstoff steht und
C für ein Element aus den Gruppen 14 und 15 steht und
D für ein Element aus den Gruppen 14 und 15 steht und
x für eine Zahl von 1 bis 4 steht und
y für eine Zahl von 1 bis 4 steht und
z für eine Zahl von 0 bis 4 steht und
w für eine Zahl von 0 bis 4 steht,
wobei A, B, C und D jeweils aus verschiedenen Gruppen oder bei gleicher Gruppe aus verschiedenen Perioden stammen, mit der Maßgabe, daß A verschieden von Aluminium ist, wenn B Kohlenstoff ist und gleichzeitig z und w gleich 0 sind, als heterogene Katalysatoren durchführt.

2. Verfahren nach Anspruch 1, dadurch gekennzeichnet, daß als Katalysatoren ein oder mehrere Verbindungen der allgemeinen Formel (III) mit nach der BET-Methode bestimmten Oberflächen von 0,1 bis 500 m²/g in Mengen von 0,5 bis 100 Gew.-% bezogen auf die Menge an Monohydroxyverbindung, bei nicht vollkontinuierlicher Fahrweise bzw. mit Belastungen von 0,1 bis 20 g Monohydroxyverbindung pro g Katalysator pro Stunde bei vollkontinuierlicher Fahrweise, eingesetzt werden.

## Claims

1. Process for the production of aryl carbonates by reacting aromatic monohydroxy compounds with phosgene or chlorocarbonates of aromatic monohydroxy compounds, characterised in that the reaction is performed at a temperature in the range from 50 to 450°C, at a pressure of 0.05 to 20 bar, in the presence of one or more compounds of the general formula (III)
AₓB_{y}C_{z}D_{w} (III),
in which
A denotes an element from groups 3 to 10, 13 and 14 of the periodic system of elements (IUPAC notation) and
B denotes an element from groups 13, 14, 15 and 16, with the exception of oxygen, and
C denotes an element from groups 14 and 15 and
D denotes an element from groups 14 and 15 and
x denotes a number from 1 to 4 and
y denotes a number from 1 to 4 and
z denotes a number from 0 to 4 and
w denotes a number from 0 to 4,
as heterogeneous catalysts, wherein A, B, C and D each originate from different groups, of if from the same group, from different periods, providing that A is different from aluminium if B is carbon and z and w are simultaneously equal to 0.

2. Process according to claim 1, characterised in that the catalysts used are one or more compounds of the general formula (III) with surface areas measured using the BET method of 0.1 to 500 m²/g in quantities of 0.5 to 100 wt.% relative to the quantity of monohydroxy compound, in the event of incompletely continuous operation, or with loadings of 0.1 to 20 g of monohydroxy compound per g of catalyst per hour in the event of completely continuous operation.

## Revendications

1. Procédé pour la préparation de carbonates d'aryles par réaction de composés aromatiques monohydroxy avec du phosgène ou des esters d'acides chlorocarboxyliques de composés aromatiques monohydroxy, caractérisé en ce que l'on réalise la réaction à une température dans le domaine de 50 à 450°C, à une pression de 0,05 à 20 bars en présence d'un ou plusieurs composés de la formule générale (III)
AₓB_{y}C_{z}D_{w} (III),
dans laquelle
A représente un élément des groupes 3 à 10, 13 et 14 de la classification périodique des éléments (nomenclature IUPAC) et
B représente un élément des groupes 13, 14, 15 et 16 à l'exception de l'oxygène et
C représente un élément des groupes 14 et 15 et
D représente un élément des groupes 14 et 15 et
x est un nombre de 1 à 4 et
y est un nombre de I à 4 et
z est un nombre de 0 à 4 et
w est un nombre de 0 à 4,
A, B, C et D provenant à chaque fois de groupes différents ou de périodes différentes pour un même groupe, à la condition que A est différent de l'aluminium lorsque B est le carbone et z et w sont simultanément égaux à 0, comme catalyseurs hétérogènes.

2. Procédé selon la revendication 1, caractérisé en ce que l'on utilise comme catalyseurs un ou plusieurs composés de la formule générale (III) présentant des surfaces spécifiques déterminées selon le procédé BET de 0,1 à 500 m²/g dans des quantités de 0,5 à 100% en poids, rapportés à la quantité de composé monohydroxy, pour un fonctionnement discontinu respectivement avec des charges de 0,1 à 20 g de composé monohydroxy par gramme de catalyseur par heure pour un fonctionnement continu ininterrompu.
